# EUROPEAN PATENT APPLICATION

(11) **EP 4 796 026 A1**
(43) Date of publication of application: **26.08.2026**
(21) Application number: 25159589.8
(22) Date of filing: 24.02.2025
(51) Int. Cl.: A61B 5/022

(54) **METHOD, DEVICE AND SYSTEM FOR DETERMINING AMBIENT ATMOSPHERIC PRESSURE**

(71) Applicant: Koninklijke Philips N.V., 5656 AE Eindhoven (NL)
(72) Inventor: KUNT, Stanislav, 5656 AG Eindhoven (NL); DUINEVELD, Paulus Cornelis, 5656 AG Eindhoven (NL)
(74) Representative: Philips Intellectual Property & Standards

(57) **Abstract**

The present invention relates to a method (200), device (10), and system (100) for determining ambient atmospheric pressure of a non-invasive blood pressure measurement system (50). The method comprises:
- obtaining a pressure difference measurement result comprising information about pressure differences over time between the inside of the NIBP measurement system and the ambient atmosphere as measured by a gauge pressure sensor of the NIBP measurement system; and
- determining the ambient atmospheric pressure based on a predetermined mathematical model, wherein the predetermined mathematical model is based on the obtained pressure difference measurement result, performance parameters of a pump of the NIBP measurement system and one or more of:
- an effective opening area of a valve and/or orifice of the NIBP measurement system if a valve and/or orifice of the NIBP measurement system is opened,
- properties of a body part of a user and an actuator of the NIBP system if the actuator is attached to the body part, and
- a volume of a rigid container if the NIBP measurement system comprises the rigid container, the rigid container at least replacing the actuator of the NIBP system.

## Description

### FIELD OF THE INVENTION

The present invention relates to a method, device, and system for determining ambient atmospheric pressure of a non-invasive blood pressure measurement system.

### BACKGROUND OF THE INVENTION

There exists a variety of hospital patient monitors with which vital body signs of a patient in a hospital can be monitored. One of these vital body signs is the blood pressure, which can be measured via a standard NIBP (Non-Invasive Blood Pressure) meter. The monitors therefore consist of a control unit that can inflate via a pump a standard blood pressure cuff (also called actuator herein) and measure from the recorded pressure hemodynamic signals such as SAP, DAP, MAP (systolic, diastolic and mean arterial pressure). There is also developed an adapted blood pressure meter with which more complicated hemodynamic parameters such as e.g. stroke volume, cardiac output etc. can be measured via inflating an adapted non-invasive blood pressure cuff.

For accurate determining of SAP, MAP, DAP with an NIBP measurement system it is important to know the compliance of a cuff. The compliance of a cuff can generally be expressed as *Cₐ* = d*Vₐ*/d*pₐ*, i.e. as the ratio of the change of the air volume supplied to the actuator (i.e. cuff) and a given change of the actuator pressure. In general, *Cₐ* is not constant but varies with the actuator pressure, as well as the size of the arm of a patient and tissue properties. Knowledge of changes of the compliance is important as it can be used to correct the blood pressure envelope.

Typically, the volume changes of the brachial artery result in a pressure change inside the actuator when the actuator is inflated. Due to the non-constant compliance, however, the pressure pulses at low actuator pressure are usually underestimated, resulting in an overestimation of the blood pressure values at low DAP, MAP and SAP, which is completely unwanted.

In US 2017/0238824 A1 the flow rate and hence volume to the actuator is measured in a complicated way which cannot be used for a standard monitor. This is particularly due to the fact that the method disclosed requires two pressure sensors and a predefined geometry/construction, such as a venturi element, introducing extra friction in the system.

There also exist simple methods using standard monitors to measure the flow rate and hence the volume towards the actuator by using only a single pressure sensor and the varying pump information (RPM and/or PWM%), so no changes to the current standard geometry/construction are required, but only a software change. Further, it is known how to deal with influences of varying (ambient) pressure, voltage and ambient temperature. However, standard monitors generally do not contain an atmospheric pressure sensor. Therefore, using standard monitors it will be difficult to accurately measure the flow rate and/or total air volume supplied to the actuator (without knowing the ambient pressure).

However, as there are also other benefits of knowing the flowrate and/or total volume towards the actuator, such as detection of blockage of an orifice, it is desirable to provide a method which allows for accurate NIBP measurements, taking effects of the ambient atmosphere into account, even in cases where only standard NIBP monitors are present.

### SUMMARY OF THE INVENTION

It is an object of the present invention to determine the ambient atmospheric pressure of a non-invasive blood pressure (NIBP) measurement system using existing sensors of said system, i.e. without requiring an additional ambient pressure sensor.

In a first aspect of the present invention a method for determining ambient atmospheric pressure of a non-invasive blood pressure, NIBP, measurement system is presented, the method comprising:
- obtaining a pressure difference measurement result comprising information about pressure differences over time between the inside of the NIBP measurement system and the ambient atmosphere as measured by a gauge pressure sensor of the NIBP measurement system; and
- determining the ambient atmospheric pressure based on a predetermined mathematical model, wherein the predetermined mathematical model is based on the obtained pressure difference measurement result, performance parameters of a pump of the NIBP measurement system and one or more of:
   - an effective opening area of a valve and/or orifice of the NIBP measurement system if a valve and/or orifice of the NIBP measurement system is opened,
   - properties of a body part of a user and an actuator of the NIBP measurement system if the actuator is attached to the body part, and
   - a volume of a rigid container if the NIBP measurement system comprises the rigid container, the rigid container at least replacing the actuator of the NIBP measurement system.

In a second aspect of the present invention a device for determining ambient atmospheric pressure of a non-invasive blood pressure, NIBP, measurement system is presented, the device comprising:
- an input unit configured to obtain a pressure difference measurement result comprising information about pressure differences over time between the inside of the NIBP measurement system and the ambient atmosphere as measured by a gauge pressure sensor of the NIBP measurement system; and
- a processing unit configured to determine the ambient atmospheric pressure based on a predetermined mathematical model, wherein the predetermined mathematical model is based on the obtained pressure difference measurement result, performance parameters of a pump of the NIBP measurement system and one or more of:
   - an effective opening area of a valve and/or orifice of the NIBP measurement system if a valve and/or orifice of the NIBP measurement system is opened,
   - properties of a body part of a user and an actuator of the NIBP measurement system if the actuator is attached to the body part, and
   - a volume of a rigid container if the NIBP measurement system comprises the rigid container, the rigid container at least replacing the actuator of the NIBP measurement system.

In a further aspect of the present invention a system for determining ambient atmospheric pressure of a non-invasive blood pressure, NIBP, measurement system is presented, the system comprising:
- the NIBP measurement system, comprising:
   - a pump configured to pump fluid from an ambient atmosphere to the inside of the NIBP measurement system,
   - a gauge pressure sensor configured to measure pressure differences between the inside of the NIBP measurement system and the ambient atmosphere, and
   - one or more of:
      - a valve and/or orifice comprising an effective opening area for releasing the fluid from the pump to the ambient atmosphere,
      - an actuator configured to be attached to a body part of a user, and
      - a rigid container configured to at least replace the actuator; and
- a device as presented herein.

In yet a further aspect of the present invention, there is provided a corresponding computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed device, system, computer program and medium have similar and/or identical preferred embodiments as the claimed method, in particular as defined in the dependent claims and as disclosed herein.

The present invention is based on the idea to accurately determine the ambient atmospheric pressure of a (standard) NIBP measurement system (i.e. around an NIBP measurement system) without adding any extra sensors, but just by using the available sensors. Hence, construction of the device and/or NIBP measurement system is simplified, space within the device and/or NIBP measurement system is saved and construction cost reductions are achieved.

The determined ambient pressure can be used as input parameter to accurately determine the flow rate and/or volume supplied to the actuator in both a standard NIBP measurement system as well as in advanced NIBP measurement systems.

The advantages of being able to measure the flow rate and/or volume to the actuator are: improving the accuracy of SAP, MAP, DAP measurements and even determining blockings of an orifice, tube, etc. of more advanced NIBP measurement systems.

To be more precise, the present invention is based on the idea to determine the ambient pressure by using the gauge pressure sensor inside an NIBP measurement system, for example in combination with knowledge of the system's effective valve diameter (and/or orifice diameter if the system comprises an orifice), properties of a body part of a user and the actuator if the actuator of the NIBP system is attached to the body part, and/or a volume of a rigid container replacing at least the actuator of the NIBP system. In other words, using the gauge pressure sensor of the NIBP measurement system there is obtained information about a pressure difference (over time) between the inside of the NIBP measurement system and the ambient atmosphere. From this pressure difference the absolute atmospheric pressure can be determined if additional parameters, particularly performance parameters, of the pump of the NIBP measurement system are provided.

For example, if the cuff/actuator of the system was removed the ambient atmospheric pressure could be determined by using the effective opening area of the valve of the NIBP measurement system used as an input parameter of a mathematical model for determining the ambient atmospheric pressure.

The fact that the predetermined mathematical model "is based on" the obtained pressure difference measurement result, performance parameters of a pump of the NIBP measurement system and one or more of an effective opening area of a valve and/or orifice, body part and actuator properties, and a volume of a rigid container does not imply that the mathematical model itself, i.e. the type of equations would be changed by the obtained pressure difference and the like. Rather, these parameters are to be understood as input parameters of the mathematical model.

The term "ambient atmospheric pressure" as used herein can be understood as the local atmospheric/environmental absolute pressure (directly) around the NIBP measurement system.

In an embodiment, the method further comprises, if the valve and/or orifice is opened, determining a steady state pressure difference from the obtained measurement result, wherein the steady-state pressure difference is represented by the pressure difference measurement result if the NIBP measurement system fails to comprise an actuator, and/or wherein the steady-state pressure difference is determined based on a function of time of the pressure differences if the NIBP measurement system comprises an actuator, wherein the predefined mathematical model comprises a function and/or a look-up table linearly relating the ambient atmospheric pressure to the steady-state pressure difference.

In other words, if the NIBP measurement system used comprises a valve and/or an orifice, the ambient atmospheric pressure may be determined by determining the effective diameter of the valve/orifice and by determining the steady state pressure difference between the inside of the NIBP measurement system and the ambient atmosphere.

In practice, to determine the ambient pressure using an NIBP measurement system with a valve, users could proceed as follows: First, the actuator (i.e. cuff) and possibly also a hose to the cuff of the system is removed. The air pumped into the system is thus almost immediately leaving the system once the pump of the NIBP measurement is operated. Then, when the pump is in operation, the pressure difference is measured (using the gauge pressure sensor) while keeping the valve and/or orifice in an opened state. Subsequently the valve/orifice may be closed. Since the actuator is removed (and thus the air pumped into the system leaves the system almost immediately) the measured pressure difference is the steady state pressure difference. Using said steady state pressure difference and knowledge of the pump parameters at a reference atmospheric pressure for the operation condition of the pump used, the ambient pressure may be determined via the predetermined mathematical model (e.g. via a linear transfer function or look up table).

However, in case removal of the actuator is not desired, the actuator may be kept connected to the NIBP measurement system. However, a steady state only establishes after a certain delay in this case because the volume of the actuator first needs to be filled. Therefore, the steady state pressure difference has to be determined based on a function of time of the pressure differences. Apart from that, the ambient pressure can still be determined in the same way as if the actuator were not present.

In any case, if it is recognized that the obtained pressure differences vary over time, steady state has not yet been achieved. Thus, the measurement result already indicates whether there is a cuff/actuator applied or not. With an actuator connected to the NIBP measurement system it will take a finite time to reach steady state, whereas in case there is no cuff applied, the steady state is obtained almost instantaneously.

In an embodiment, the method further comprises determining whether the function is an appropriate mathematical model by determining whether the ratio of the steady-state pressure difference and the ambient atmospheric pressure is higher than 0.005 and equal or smaller than 0.15, particularly whether the ratio of the steady-state pressure difference and the ambient atmospheric pressure is higher than 0.02 and equal or smaller than 0.1.

In a further embodiment, the method further comprises determining one or more further steady-state pressure differences, and determining a mean steady-state pressure difference by averaging the plurality of steady-stated differences, wherein the ambient atmospheric pressure is determined based on the mean steady-state pressure difference.

This way, deviations in individual measurements can be compensated.

In another embodiment, the effective opening area of the valve and/or orifice is determined by measuring a deflation time of the valve and/or orifice for reducing an initial pressure to a final pressure in a predefined deflation volume and by relating the measured deflation time to the effective valve and/or orifice diameter based on a predetermined lookup table.

Thus, the valve opening is not measured directly; instead, its effective opening is determined based on the properties of the valve. This approach allows for a precise assessment of the valve's opening size.

In an embodiment, the method further comprises, if the actuator of the NIBP system is attached to the body part and/or if the NIBP measurement system comprises the rigid container, determining a temporal pressure difference gradient based on the obtained pressure difference measurement result, wherein the ambient atmospheric pressure is determined based on the temporal pressure difference gradient and the predefined mathematical model.

In an embodiment, the predefined mathematical model comprises a function and/or a look-up table, linearly relating the ambient atmospheric pressure to the temporal pressure difference gradient.

In general, a linear model is easier to implement and is computationally efficient. It allows for quick calibration and adaptation to different (environmental) conditions.

In yet another embodiment, the performance parameters of the pump of the NIBP measurement system are functions of one or more of the ambient atmospheric pressure, the ambient atmospheric temperature, a voltage applied to the pump, and pulse width modulation percentage of a motor of the pump.

The performance parameters of the pump of the NIBP measurement system particularly refer to parameters required to describe the flow rate at a given PWM%, voltage, reference ambient pressure and reference ambient temperature. In particular, the parameters *a, b, c,* and *d* described below in equation (3) are referred to as performance parameters of the pump herein. For example, in general, the pump performance is influenced by voltage that is applied to the pump. For an NIBP measurement system connected to a power supply, the voltage will be reasonably consistent, however, for a battery drive system, the voltage may change and thus it may be useful to measure said quantity.

The ambient temperature can be understood as the local atmospheric/environmental absolute temperature (directly) around the NIBP measurement system.

In an embodiment of the method, the performance parameters of the pump of the NIBP measurement system are determined based on reference performance parameters of the pump of the NIBP measurement system at reference ambient atmospheric pressure and reference ambient atmospheric temperature.

In other words, even if current performance parameters of the pump of the NIBP measurement system are not known, they can be determined via reference values.

In a further embodiment, the ambient atmospheric temperature is obtained from a temperature sensor of the NIBP measurement system.

Obtaining temperature values directly from an integrated temperature sensor allows to obtain precise temperature values und thus allows for a more precise ambient pressure measurement result.

In another embodiment, the method further comprises determining a flow rate and/or volume supplied to an actuator of the non-invasive blood pressure measurement system based on the determined ambient atmospheric pressure.

The flow rate can be understood as a measure of the volume or fluid (i.e. gas or liquid, typically air in the NIBP measurement system) moving from the pump to the actuator per unit of time.

In an embodiment of the system, the system further comprises a temperature sensor configured to measure ambient temperature.

### BRIEF DESCRIPTION OF THE DRAWINGS

These and other aspects of the invention will be apparent from and elucidated with reference to the embodiment(s) described hereinafter. In the following drawings:
Fig. 1 shows a schematic diagram of a conventional NIBP measurement system the present invention makes use of;
Fig. 2 shows a flow chart of a first embodiment of a method according to the present invention;
Fig. 3 shows a diagram of the Q-H curve of a pump of the NIBP measurement system;
Fig. 4 shows, as an example, the average flow rate of a batch of pumps operated in the voltage range from 9 to 14.8 V;
Fig. 5A shows the parameter *a* as a function of Δ*V.*
Fig. 5B shows the parameter *b* as a function of Δ*V*.
Fig. 5C shows the parameter *c* as a function of Δ*V*.
Fig. 5D shows the parameter *d*, i.e. *Q_{vol,max}*, as a function of Δ*V.*
Fig. 6 shows a diagram of a relation between the expected (i.e. calculated) pressure difference Δ*p* as a function of the effective valve diameter *dᵥₐₗᵥₑ* for different ambient atmospheric pressures;
Fig. 7 shows a diagram of Δp as a function of the ambient atmospheric pressure for different effective valve diameters;
Fig. 8 shows a diagram of a pressure difference in a box of 250 ml comprising the valve as only opening as a function of time;
Fig. 9 shows a diagram of the "variation" in effective valve diameter with allowed variation in release time (for the above given parameters and several box sizes);
Fig. 10 shows the results of the mathematical model presented herein and actual measurements;
Fig. 11 shows a flow chart of a second embodiment of a method according to the present invention;
Fig. 12 shows a diagram of the change of pressure with time for different valve sizes;
Fig. 13A shows a diagram of the pressure difference over time for *dᵥₐₗᵥₑ* = 0.95 *mm* as full solution and as a simple fit,
Fig. 13B shows a diagram of the pressure difference over time for *dᵥₐₗᵥₑ* = 0.55 *mm* as full solution and as a simple fit;
Fig. 14 shows a flow chart of a third embodiment of a method according to the present invention;
Fig. 15 shows a diagram of (the increase of) the pressure difference with time in a rigid container according to experiment (dashed line) and the theory (solid line) at two different ambient pressure conditions;
Fig. 16 shows a flow chart of a fourth embodiment of the method according to the present invention;
Fig. 17 shows an embodiment of a device according to the present invention; and
Fig. 18 shows an embodiment of a system according to the present invention.

### DETAILED DESCRIPTION OF EMBODIMENTS

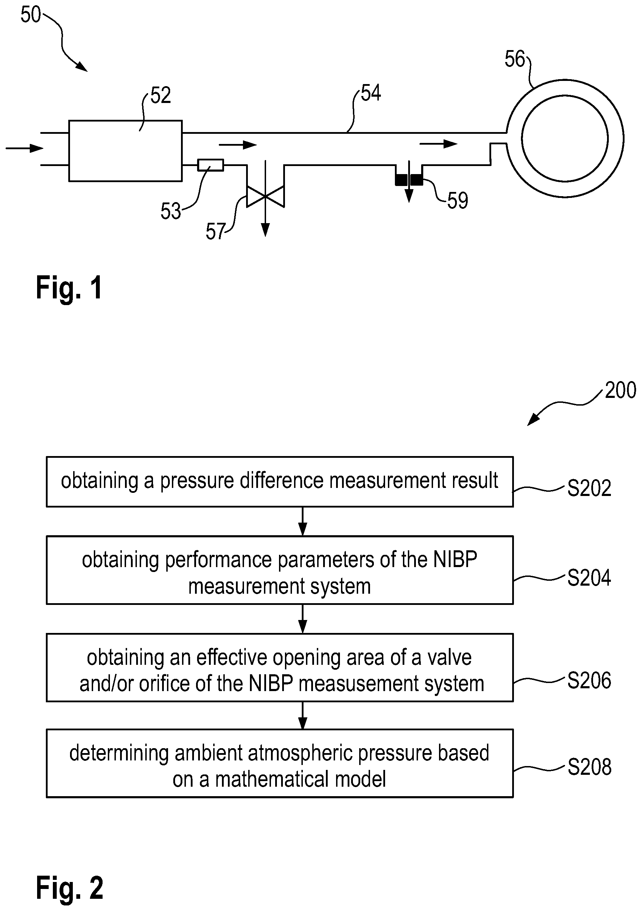

Fig. 1 shows a schematic diagram of a conventional NIBP measurement system 50, the present invention makes use of. The NIBP measurement system 50 comprises a pump 52, a gauge pressure sensor 53, a hose 54 and a cuff 56 (which acts as the actuator). Furthermore, the NIBP measurement system 50 comprises a valve 57 and an orifice 59. The pump 52 is configured to inflate the cuff 56 by sucking in air from the ambient atmosphere and pushing the air through the hose 54, applying pressure to a patient's body part (usually an arm, wherein the cuff 56 is configured to surround the arm). Once the target pressure is reached, the valve 57 gradually releases air, allowing the system 50 (more particularly an analysis unit or the like being also part of the system 50 or connected to it) to detect oscillations in pressure. These oscillations are analyzed to determine systolic and diastolic blood pressure, for example. To detect the pressure difference between the pressure inside of the NIBP measurement system 50 and the ambient atmosphere, the gauge pressure sensor 53 is used. The hose 54 serves as the connection between the components, ensuring controlled airflow throughout the measurement process.

In the NIBP measurement system 50 shown in Fig. 1, for simplicity, only a single valve 57 is included, but in general the NIBP measurement system 50 could likewise comprise a combination of multiple valves 57 (and orifices 59).

The cuff 56, i.e. actuator, may generally comprise any suitable (non-invasive) actuator (for use in an NIBP measurement system) such as for example, a bladder cuff actuator, a wrist cuff actuator, or a finger cuff actuator.

Fig. 2 shows a flow chart of a first embodiment of a method 200 according to the present invention. In this embodiment, in the NIBP measurement system 50 used, the cuff 56 (and possibly (parts of) the hose 54) are not present, i.e. removed.

Ambient air at unknown ambient pressure (and possibly unknown ambient temperature) is transported by the pump 52 (via the hose 54) through the valve 57, which is kept open, and through the optional orifice 59 (with a much smaller cross sectional area than the at least one valve opening). As either the cuff 56 or the whole connecting hose 54 can be removed the air cannot flow through said parts. At the point of removal no opening to the atmosphere shall emerge. In a first step S202 of the method 200 a pressure difference measurement result is obtained, i.e. there is obtained information about pressure differences over time as measured by the gauge pressure sensor 53. In this setup (with no cuff 56 included) the pressure difference Δ*p* (with respect to the ambient pressure) will soon (in fact almost instantaneously) reach a steady state value. As there is no flow rate to the actuator (because there is no actuator in the NIBP measurement system), ${Q}_{vol , pump} = {Q}_{vol , valve}$

Given this knowledge, and information about performance parameters of the pump 52 of the NIBP measurement system 50 as well as information about the effective opening area of the valve 57 (and orifice 59) the ambient atmospheric pressure can be determined. Hence, in a second step S204 performance parameters of the pump 52 of the NIBP measurement system 50 are obtained and in a third step S206 information about the effective opening area of the valve 57 is obtained. In a fourth step S208, making use of a mathematical model based on the given information, the ambient atmospheric pressure can be determined. How the mathematical model works (and how it is established) will be explained in the following:
For small Δ*p* the flow rate through the valve 57 can be described with incompressible flow theory. Hence, the volumetric flow rate though the valve 57 can be reasonably described by ${Q}_{vol , valve} = \sqrt{\frac{Δ {pRT}_{a}}{\left({p}_{a}+Δp\right) k}} {A}_{valve} ,$ wherein *Aᵥₐₗᵥₑ* is the effective opening area of the valve 57 (with ${A}_{valve} = π / 4 {d}_{valve}^{2}$, where *dᵥₐₗᵥₑ* is the effective valve diameter) and *k* is a resistance parameter, which may be set to *k* =1.1, *R* is the gas constant for air *R* = 8.31/0.029 = 287 *J*/(*Kg K*) and *Tₐ* is the (absolute) ambient atmospheric temperature. Effects of the presence of the orifice 59 will be ignored for the time being.

The volumetric flow rate through the pump 52 can generally be described as follows: $\begin{matrix}{Q}_{vol , valve} = a \left({p}_{a},PWM%,ΔV,{T}_{a}\right) Δ {p}^{3} + b \left({p}_{a},PWM%,ΔV,{T}_{a}\right) Δ {p}^{2} \\ + c \left({p}_{a},PWM%,ΔV,{T}_{a}\right) Δ p + d \left({p}_{a},PWM%,ΔV,{T}_{a}\right) ,\end{matrix}$ wherein *d = Q_{vol,max},* i.e., the maximum volumetric flow rate the pump 52 can deliver for a given ambient atmospheric pressure *pₐ*, a given pulse width modulation percentage *PWM%* of the pump 52 of the NIBP measurement system 50, a given voltage Δ*V* applied to the pump 52 and a given ambient temperature *Tₐ*. It has to be noted that the volumetric flow rate is the flow rate at local pressure (which is the ambient atmospheric pressure plus the pressure difference generated by the pump) and temperature, while a reference flow rate is a flow rate at reference temperature and pressure. Apart from the parameter *d,* also the parameters *a, b,* and c depend in principle on the ambient atmospheric pressure *pₐ*, pulse width modulation percentage *PWM%* of the pump 52 of the NIBP measurement system 50, voltage *ΔV* applied to the pump and ambient (atmospheric) temperature *Tₐ*. In the given setting PWM% is known, such that the pump performance parameters *a, b, c* and *d* are known at a reference atmospheric pressure, reference temperature and applied voltage. Preferably, the pump 52 is operated at its full capacity, i.e. 100% PWM, but in principle it is also possible to determine *a, b,* c and *d* at the reference atmospheric pressure and temperature for other PWM%. The correction of the parameters *a, b, c* and *d* with respect to voltage is also known by those skilled in the art. In principle the correlation for each parameter at the reference ambient pressure and temperature is given as simple linear or quadratic relations with voltage.

In general, it should be noted that simpler descriptions than (3) are possible such as a quadratic relation. Solving (3) for the maximum pressure the pump 52 can deliver, one gets as the only real (non-imaginary) solution (the solution follows from solving Δ*p* from (3) for *Q* = 0; typically, a pump curve has a maximum flow rate at Δ*p*=0 and a maximum Δ*p* at zero flow rate): $\begin{matrix}Δ {p}_{max} = \frac{- b}{3 a} - \frac{{2}^{\frac{1}{3}} \left(3ac-{b}^{2}\right)}{3 a {\left(-2{b}^{3}+9abc-27{a}^{2}d+\sqrt{4 {\left(-{b}^{2}+3ac\right)}^{3} + {\left(-2{b}^{3}+9abc-27{a}^{2}d\right)}^{2}}\right)}^{\frac{1}{3}}} \\ + \frac{{\left(-2{b}^{3}+9abc-27{a}^{2}d+\sqrt{4 {\left(-{b}^{2}+3ac\right)}^{3} + {\left(-2{b}^{3}+9abc-27{a}^{2}d\right)}^{2}}\right)}^{\frac{1}{3}}}{3 a ⋅ {2}^{\frac{1}{3}}}\end{matrix}$

For finding the scaling for the parameters *a, b, c* and *d,* the following conditions can be used:
i. The Δ*Pₘₐₓ* scales linearly with the ambient pressure. In fact, it was found from experiments that $Δ {p}_{max , n} = Δ {p}_{max , ref} \frac{{p}_{a}}{{p}_{a , ref}} \sqrt{\frac{{T}_{a , ref}}{{T}_{a}}} ,$ wherein the designation "ref" refers to reference values.
ii. ${Q}_{vol , max , n} = {Q}_{vol , max , ref} \sqrt{\frac{{T}_{a , ref}}{{T}_{a}}}$
iii. The gradient $\frac{d {Q}_{p}}{dΔ {p}_{cuff}} \left(Δ{p}_{cuff}=0\right)$ remains equal (at maximum flow rate, i.e. at $\left.Δ{p}_{cuff}=0\right) , hence {c}_{n} = {c}_{ref} \frac{{p}_{a , ref}}{{p}_{a}} \frac{{T}_{a}}{{T}_{a , ref}}$
iv. The gradient ${\left.\frac{d {Q}_{p}}{dΔ {p}_{cuff}}\left(Δ{p}_{max}\right)\right|}_{old} = {\left.\frac{d {Q}_{p}}{dΔ {p}_{cuff}}\left(Δ{p}_{max}\right)\right|}_{new}$, this gives *aₙ =* $\frac{2}{3} {b}_{n} \frac{1}{Δ {p}_{max , n}} + {a}_{ref} {\left(\frac{Δ {p}_{max , ref}}{Δ {p}_{max , n}}\right)}^{2} \frac{{p}_{a , ref}}{{p}_{a}} \frac{T}{{T}_{ref}} - \frac{2}{3} {b}_{ref} \frac{Δ {p}_{max , ref}}{Δ {p}_{max , n}^{2}} \frac{{p}_{a , ref}}{{p}_{a}} \frac{{T}_{a}}{{T}_{a , ref}}$
v. *bₙ* is calculated in from Δ*p_{max,new}*(*aₙ, bₙ, cₙ, Q_{p,max,new}*) *=* $\frac{{p}_{a}}{{p}_{a , ref}} \sqrt{\frac{{T}_{a , ref}}{{T}_{a}}} Δ {p}_{max , ref} \left({a}_{ref}, {b}_{ref}, {c}_{ref}, {Q}_{p , max , ref}\right)$

The parameters *aₙ, bₙ, cₙ* and *d*ₙ *= Q_{p,max,n}* can be found at any ambient pressure and temperature when reference values *a_{ref}, b_{ref}, c_{ref}* and *d*_{ref} = *Q_{p,max,ref}* are known. The scaling with ambient pressure follows from basic physics and the scaling with temperature for Δ*p_{max,n}* and *Q_{p,max,n}* from experiments. In principle it is possible to generalize this relation to ${\left(\frac{{T}_{a , ref}}{{T}_{a}}\right)}^{α}$. *bₙ* needs to be solved numerically.

It can be shown that there is approximately a linear relation between the measured Δ*p* and the unknown ambient pressure *p*ₐ. In principle this can be done by numerically solving equation (1), with use of equations (2) and (3) and the procedure to determine the unknown pump parameters *aₙ, bₙ, cₙ* and *d*ₙ*= Q_{p,max,n}.*

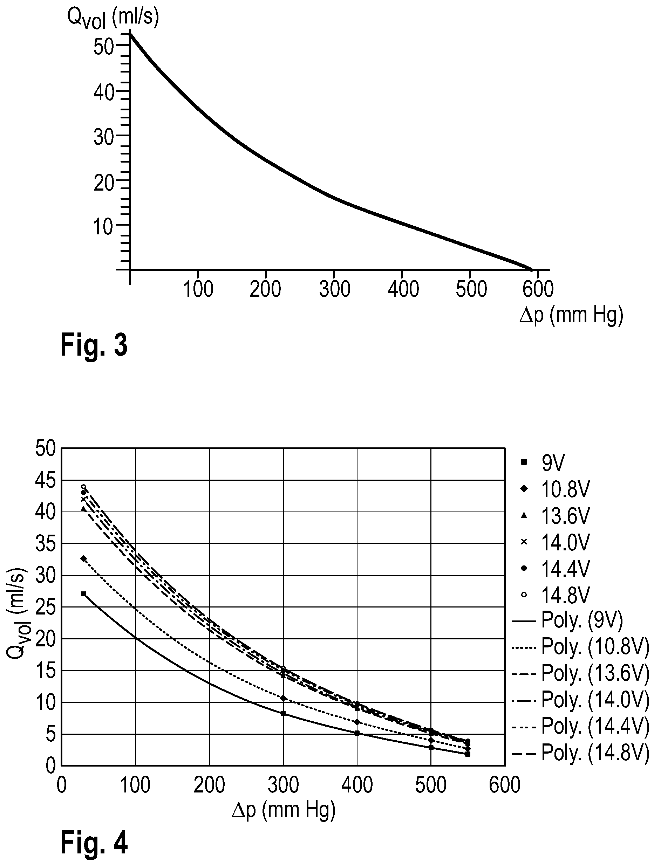

As Δ*p* is relatively small, the relation between flow rate and pressure simplifies. This can be seen from Fig. 3, for example.

Fig. 3 shows a diagram of the Q-H curve of a mean performing pump by operated at 100% PWM and 14.4 V, at. *p*_{a,ref} = 0.95 bar and *T_{ref}* ≈ 23 °C. The Q-H-curve describes the relationship between flow rate (Q) and pressure head (H), particularly concerning the air flow through the pump-hose(-cuff) circuit, the flow rate Q indicating how much air flows through the system per unit of time, typically measured in millilitres per second (ml/s) and the pressure head H representing the pressure built up in the NIBP measurement system 50 (i.e. hose, cuff), typically measured in mm Hg (with respect to the ambient pressure).

For small Δ*p* the complicated cubic relation (see equation (3) above) can be simplified to a linear relation: ${Q}_{vol , pump} \approx {Q}_{max} \left({T}_{a}\right) - {c}_{n} Δ p$

Using of the scaling conditions (2) and (3) ${Q}_{vol , pump} \approx {Q}_{max , ref} \sqrt{\frac{{T}_{a , ref}}{{T}_{a}}} - {c}_{ref} \frac{{p}_{a , ref}}{{p}_{a}} \frac{{T}_{a}}{{T}_{a , ref}} Δ p$

Balancing with (2) gives: $\begin{matrix}{Q}_{max , ref} \sqrt{\frac{{T}_{a , ref}}{{T}_{a}}} - {c}_{ref} ⋅ {p}_{a , ref} \frac{{T}_{a}}{{T}_{a , ref}} \frac{Δ p}{{p}_{a}} = \sqrt{\frac{Δ {pRT}_{a}}{\left({p}_{a}+Δp\right) k}} {A}_{valve} \\ = \sqrt{\frac{\frac{Δ p}{{p}_{a}} {RT}_{a}}{\left(1+\frac{Δ p}{{p}_{a}}\right) k}} {A}_{valve}\end{matrix}$

From (7) it can be observed that: $\frac{Δ p}{{p}_{a}} = f \left({Q}_{max , ref}, {T}_{a}, {T}_{a , ref}, {c}_{ref}, {p}_{a , ref}, k, {A}_{valve}\right)$

Hence there is a linear relationship between the unknown ambient pressure *pₐ* and the measured pressure difference Δ. Here, *Q_{max,ref} and c_{ref}* can be functions of PWM% and the voltage ΔV.

Equation (7) can be solved for $\frac{Δ p}{{p}_{a}}$. It is a solution of a cubic equation, which can be solved analytically according to standard mathematical methods. Comparing the analytical method with the full numerical method by solving *pₐ* as a function of Δ*p* for given *Q_{max,ref}, Tₐ, T_{a,ref}, c_{ref}, p_{a,ref}, k, Aᵥₐₗᵥₑ* it can be noticed that the simplified analytical solution is equal to the full numerical solution when the complete Q-H curve (described by the cubic relation (3) (being valid over the complete Δ*p*-range)) of the pump is used when the pressure difference is relatively small, i.e. $\frac{Δ p}{{p}_{a}} \approx < 0.08$.

Hence, if the (steady state) pressure difference Δ*p* is given along with parameters of the NIBP measurement system 50 such as *Q_{max,ref} and c_{ref}* and information about the effective opening area of the valve 57, *Aᵥₐₗᵤₑ,* the (absolute) ambient atmospheric pressure *pₐ* can be calculated (i.e. determined) as explained above. As already mentioned, however, *Q_{max,ref} and c_{ref}* can be functions of PWM% and the voltage Δ*V*.

The influence of the voltage on the flow rate (see eq. (3)) of the NIBP measurement system 50 is described in the following. Typically, the influence of the voltage Δ*V* on the four parameters *a, b, c* and *d* of eq. (3) at a reference ambient pressure and temperature may be described by: $p \left(ΔV\right) = {a}_{1} Δ {V}^{3} + {a}_{2} Δ {V}^{2} + {a}_{3} Δ V + {a}_{4}$

This expression was found to be valid when the voltage range is large, e.g. from 8V to 16V and can be simplified to a simpler expression when the voltage range is more limited: $p \left(ΔV\right) = {a}_{3} Δ V + {a}_{4}$

Fig. 4 shows, as an example, of the average flow rate of a batch of pumps operated in the voltage range from 9 to 14.8 V (with 725 mm Hg ambient pressure and 23°C at 100% PWM). It is found that the influence of Δ*V* is especially strong at low Δp, which also applies if PWM% is not 100%.

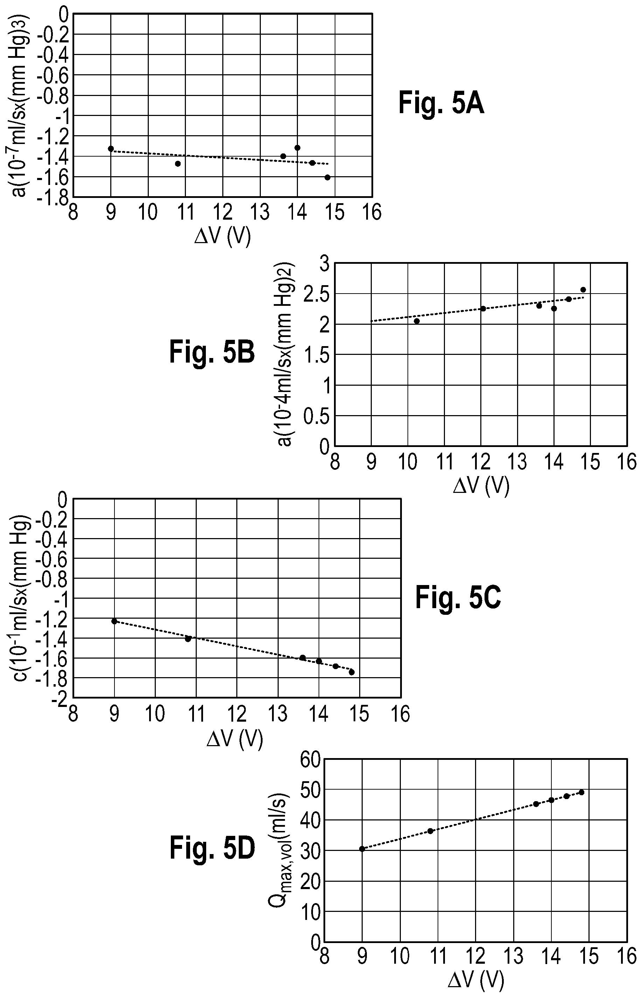

Fig. 5A shows the parameter *a* as a function of Δ*V.*

Fig. 5B shows the parameter *b* as a function of Δ*V*.

Fig. 5C shows the parameter *c* as a function of Δ*V*.

Fig. 5D shows the parameter *d*, i.e. *Q_{vol,max},* as a function of Δ*V.*

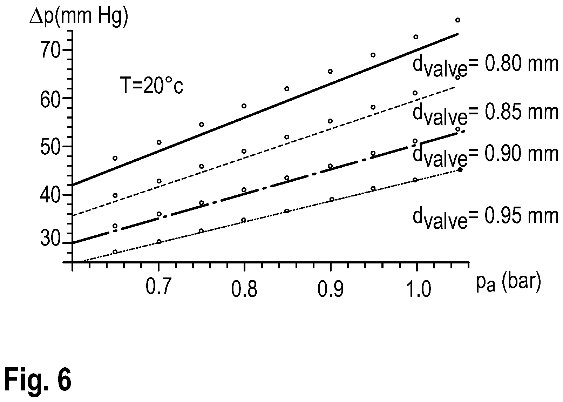

Fig. 6 shows a diagram of a relation between the expected (i.e. calculated) pressure difference Δ*p* as a function of the effective valve diameter *dᵥₐₗᵥₑ* for different ambient pressures for a pump operated at 14.4 V and at 100% PWM (wherein the pressure difference is calculated using the above mentioned formulas). From Fig. 6 it can be seen that the effective valve opening area should not be chosen too large, as otherwise Δ*p* would be too small, which may give issues with signal-to-noise ratio.

Preferably the effective valve diameter is smaller than 2.5 mm and more preferably it is smaller than 1.2 mm. When multiple valves 57 are being used potentially in combination with an orifice 59, the total effective valve diameter can be written as: ${d}_{valve , effective} \approx \sqrt{{d}_{valve , 1}^{2} + {d}_{valve 2}^{2} + {d}_{valve , 3}^{2} + ⋯} .$

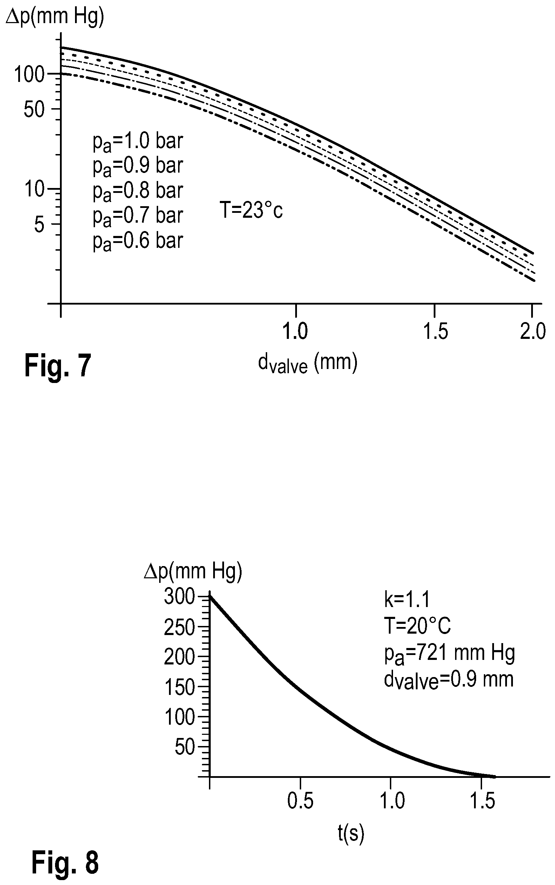

Fig. 7 shows a diagram of Δp as a function of atmospheric pressure for effective valve diameter of 0.8, 0.85 and 0.95mm. **In** other words, Fig. 7 shows the operating range for Δ*p* as a function of the atmospheric pressure for different effective valve diameters. The solid line represents the analytical solution and the dots represent full numerical calculations for a pump operated at 14.4 V and an ambient temperature of 20°.

As can be seen from Fig. 7 the complicated numerical solution still gives a linear response of Δ*p* with the atmospheric pressure. Preferably the effective diameter is in the range of 0.9 mm or larger as this has the advantage that the simple analytical solution can very well predict the atmospheric pressure based on the measured Δ*p*.

From Figs. 4 and 5 and the analytical model presented above it is obvious that the effective valve diameter should be accurately known. **In** fact, the effective valve diameter is an important parameter. The effective valve diameter can be measured, for example, by recording for each valve 57 of the NIBP measurement system 50 the deflation time to reduce the pressure in a box of 250 ml from 300 mm Hg to 10 mm Hg. A conventional deflation time (duration) is 1.8 ± 0.4 s. From this deflation time the effective valve diameter can be determined as follows:
The temporal change of the pressure difference Δ*p_{b}* in the box can be described with: $\frac{{V}_{box}}{{RT}_{a}} \frac{dΔ {p}_{b}}{d t} = - {\dot{m}}_{valve} ,$ wherein *V_{box}* is the volume of the box. The mass flow rate through the valve 57 can be found from (2) to be ${\dot{m}}_{valve} = \frac{{p}_{a} + Δ {p}_{b}}{{RT}_{a}} \sqrt{\frac{Δ {p}_{b} {RT}_{a}}{\left({p}_{a}+Δ{p}_{b}\right)}} {πd}_{valve}^{2} .$

Inserting (11) in (10) and solving the differential equation gives for the pressure difference in the box $Δ {p}_{b} \left(t\right) = {p}_{a} {\left(sinh\left[\frac{1}{2}\left(-\sqrt{\frac{{RT}_{a}}{k}}\frac{\frac{π}{4} {d}_{valve}^{2}}{{V}_{box}}⋅t+{sinh}^{- 1}\left[\sqrt{\frac{Δ {p}_{0}}{{p}_{a}}}\right]\right)\right]\right)}^{2}$ Here Δ*p*₀ is the initial pressure in the box.

Fig. 8 shows a diagram of a pressure difference in a box of 250 ml comprising the valve 57 as only opening as a function of time, wherein *k =* 1.1, *Tₐ = 20°C, pₐ* = 721 mm *Hg* and *dᵥₐₗᵥₑ =* 0.9 *mm.* As can be seen from Fig. 8 the duration, i.e. time it takes, to release the air through the valve 57 out of the box with an initial pressure of 300 mm Hg and a final pressure of 10 mm Hg roughly amounts to 1.4 s, which is consistent with the conventional deflation time of 1.8 ± 0.4 s.

Given the above mentioned theoretical model the release time can be linked to the effective valve diameter (with prescribed value of *k*)*.*

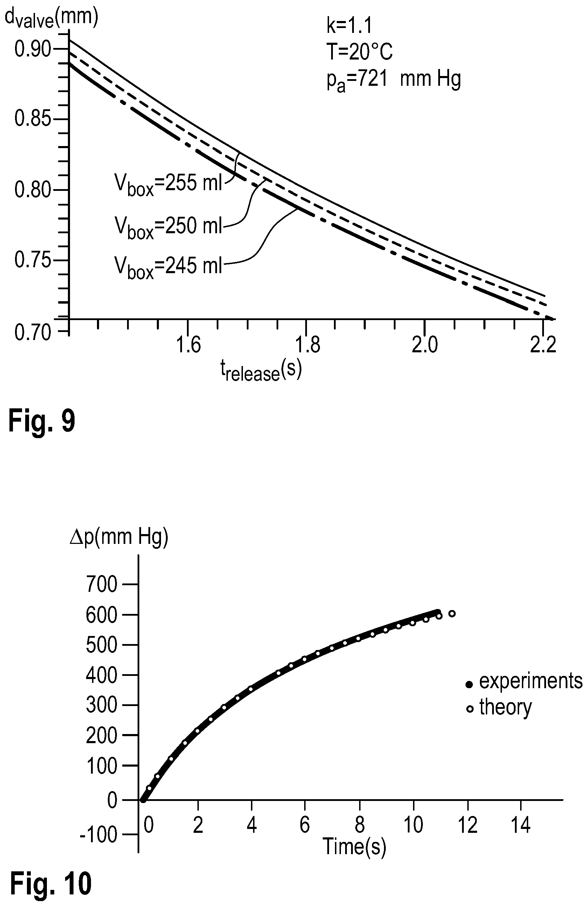

Fig. 9 shows a diagram of the variation in effective valve diameter with allowed variation in release time (for the above given parameters and several box sizes).

Actual measurements of the release time show that the mean release time amounts to *t* = 1.87 s with a standard deviation of 0.047 s. So, most valves are close to the mean. At the mean release time the effective valve diameter is, based on the above model, 0.776 mm. However, among 4060 samples the minimum release time was 1.73 s and the maximum release time was 2.18 s. Thus, it is important to use the release time to determine the effective valve diameter. Based on the model and/or experiments it is possible to make simple look up tables where the effective valve diameter is determined from the release time, wherein temperature, atmospheric pressure and actual box volume are used as input parameters.

For another conventional NIBP measurement system 50 comprising another valve 57 (and additionally an orifice 59) the mean release time was measured to be 3.435 s with a standard deviation of 0.0316 s. Based on the given model this results in an effective valve diameter of approximately 0.573 mm, resulting in a total mean effective valve diameter of 0.965 mm (as calculated using formula (9) above), which is in the preferred range.

For yet another NIBP measurement system 50 with an effective valve diameter of 0.153 mm, an additional orifice would increase the effective diameter to 0.977 mm, so only a very small effect.

In general, the "box" mentioned does not necessarily have to be a cuboid-shaped container. The term box ultimately refers to any rigid container, meaning a container that does not deform, at least under the specified pressure influences.

Furthermore, it has to be noted that in general, there exists also some influence of the pump performance; this performance can vary from pump to pump. In a similar way with the valve it is possible to measure each pump on the time to increase the pressure in a box and use this time to determine the constants *a, b, c*, and *d* at reference conditions. $\frac{d Δ p}{dt} = \frac{{T}_{b}}{{T}_{p}} \frac{{p}_{a} + Δ p}{\left({V}_{b}+{V}_{t}\right)} {Q}_{vol} \left(Δp,T,{p}_{a},ΔV,PWM%\right)$

Fig. 10 shows the results of the above given mathematical model (theory) and actual measurements. In particular, Fig. 10 shows a diagram of the pressure difference in a box of 250 ml with 3 ml of internal tubing volume over time (Pump: H016R pump driven at 13.6V at 100% PWM) a measured in experiments (dotted line) and as determined theoretically.

It is possible to determine the gradient of the pressure difference at several time intervals and solve equation (13) at least for the constants c and *d* (*Q*ₘₐₓ). In fact, one can easily see that from $\frac{d Δ p}{dt}$ at *t* = 0, *Q*ₘₐₓ and *c* can be determined. Other options to determine the parameters is by a standard Q-H measurement of the pump.

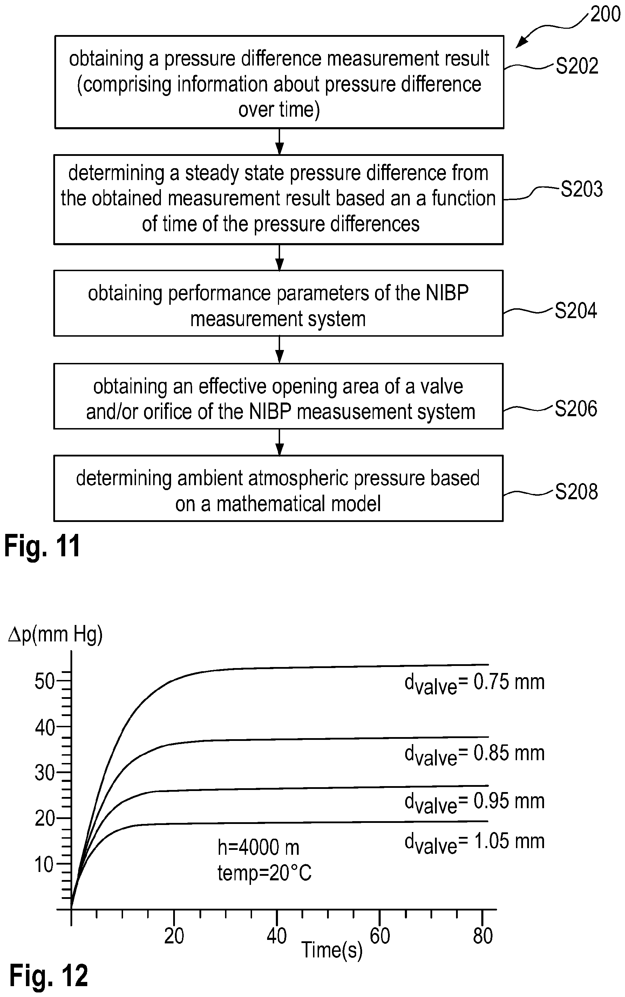

Fig. 11 shows a flow chart of a second embodiment of a method 200 according to the present invention. In this embodiment, in the NIBP measurement system 50 used, the cuff 56 is present, i.e. not removed.

Ambient air at unknown ambient pressure (and possibly unknown ambient temperature) is transported by the pump 52 (via the hose 54) through the valve 57, which is kept open, and through the optional orifice 59 (with a much smaller cross-sectional area than the at least one valve opening). As the cuff 56 (i.e. actuator) is present, the air pumped into system 50 will eventually also inflate the cuff 56 until a steady state is reached.

In a first step S202 of the method 200 a pressure difference measurement result is obtained, i.e. there is obtained information about about pressure differences over time as measured by the gauge pressure sensor 53. The initially measured pressure differences - in this case - do not correspond to steady state pressured differences. Therefore, in a second step S203, based on the obtained measurement result, a steady state pressure difference is determined. In fact, when the cuff 56 is on the arm it takes some time until the steady state pressure difference Δ*p* is obtained.

The following steps S204 to S208 are the same as for the first embodiment of the method 200 presented in accordance with Fig. 2.

Fig. 12 shows a diagram of the change of pressure with time for different valve sizes at 4000 m altitude and for worst case actuator elastance (LA2-37 arm), for a pump operated at 14.4 V. A larger valve diameter will decrease the time to reach steady state. Furthermore, in general the time to inflate depends heavily on the type of arm in the cuff.

Although, as can be seen from Fig. 12, the time to reach steady state is not that large, typically 15 seconds, it is worthwhile to reduce the time to accurately predict the steady state pressure. In other words, in a clinical setting where every procedure has to be performed efficiently, even 15 seconds can be too long. Hence, there is a need for being able to predict the steady state pressure difference before the steady state pressure difference is reached.

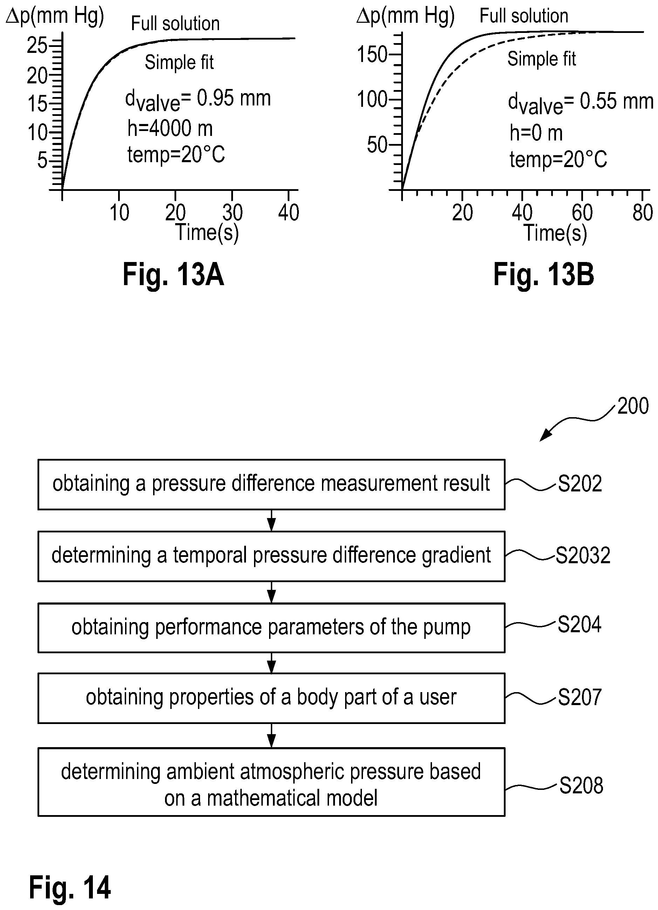

In Fig. 13A and 13B the exact solution of the differential equation (13) above (as "full solution"; solid line) and a very simple fitting function $Δ p = Δ {p}_{steady} \left(1-{e}^{- kt}\right) ,$ ("simple fit"; dashed line) is plotted for two different valve diameters, respectively. To be more precise, Fig. 13A shows a diagram of the pressure difference over time for *dᵥₐₗᵥₑ* = 0.95 *mm* as full solution to the equation (13) and as a simple fit and Fig. 13B shows a diagram of the pressure difference over time for *dᵥₐₗᵥₑ* = 0.55 *mm* as full solution to the equation (13) and as a simple fit. For this plot, worst case elastance is assumed. When the effective valve diameter is sufficiently large (i.e. in Fig. 13A), it appears that the simple fit is very accurate (the solid line representing the fitted values), hence the steady state value of the pressure can be very easily and thus quickly determined, far from the time to reach steady state. When the effective diameter is too small, the simple model is not able to effectively model the exact curve as can be seen from Fig. 13B. Therefore, there is an optimum range of the effective valve diameter. It should be small enough to have a sufficiently high steady state Δ*p,* while it should be large enough to have a sufficiently fast response and to make it simple to predict the steady state pressure with a simple fitting function. The experimental results shown in Figs. 11A and 11B, respectively, are shown for a pump operated at 100% PWM and at 14.4 V. In principle this solution is applicable for any pump that is used.

Preferably, $0.005 < \frac{Δ p}{{p}_{a}} \approx < 0.15$ and more preferably $0.02 < \frac{Δ p}{{p}_{a}} \approx < 0.1$.

Note that the steady state pressure difference, once reached, may be measured multiple times to average out variation, by determining the mean steady state pressure difference. With a sampling rate of 1 kHz it is possible to obtain easily more than 100 data points, which offers the opportunity to reduce the measurement error of the mean steady state pressure difference with at least of factor of ten.

When the steady state pressure difference value is obtained via fitting of measurement data to equation (14) above, it is possible to repeat this procedure several times, also to reduce the measurement error, when required.

In principle it is not even required to measure the (steady state) pressure difference as such to determine the atmospheric pressure. The ambient pressure can also be determined from the initial gradient of the pressure difference. In fact, it is also possible to determine the ambient pressure without knowing the valve properties.

One method to do this is by inflating an actuator e.g. on the arm of the user, which requires knowledge of the actuator and the arm properties, as will be explained.

Fig. 14 shows a flow chart of a third embodiment of a method 200 according to the present invention. In this embodiment, the actuator/cuff 56 of the NIBP measurement system 50 used may be attached to a body part of the user.

Concerning the approaches explained with respect to Figs. 2 and 9, an analytical expression for the steady state pressure difference in case the steady state pressure difference is relatively small has been found. This analysis can be extended to the initial linear pressure difference increase with time. From analysis it can be shown that for very small Δp: $\frac{dΔ p \left(t\right)}{d t} \approx \left({T}_{pump}, {T}_{ref , cuff}, {T}_{ref , pump}\right) \frac{{p}_{a}}{{p}_{ref , cuff}} {Q}_{max , ref , vol} ⋅ {a}_{v} ,$ wherein *f*(*Tₚᵤₘₚ, T_{ref,cuff}, T_{ref,pump}*) is a function depending on the temperature measured in the pump *Tₚᵤₘₚ* (as measured by a temperature sensor inside the pump, for example), the temperature at which the actuator/cuff properties have been measured, *T_{ref,cuff},* and the reference temperature, *T_{ref,pump},* where the pump performance has been measured. *pₐ* is the ambient atmospheric pressure to be determined, while *P_{ref,cuff}* is the reference absolute pressure where the cuff properties have been measured. *Q_{max,ref,vol}* is the maximum volumetric flow rate the pump delivers at the reference pressure and reference temperature of the pump. Finally, *aᵥ* combines properties of the cuff and patient arm properties. At the reference temperature and pressure where the actuator properties are measured the actuator pressure difference can be described as a quadratic function of the air volume (at the reference pressure and temperature) in the cuff, *V_{cuff}* as: $Δ {p}_{act} = {a}_{v} {V}_{act} + {b}_{v} {V}_{act}^{2}$

The parameters *aᵥ* and *bᵥ* depend on the actuator size and the arm size and compressibility.

The function *f*(*Tₚᵤₘₚ, T_{ref,cuff}, T_{ref,pump}*) can be described in a generic way as $f \left({T}_{pump}, {T}_{ref , cuff}, {T}_{ref , pump}\right) \approx \frac{{T}_{ref , cuff}}{{T}_{pump}} {\left(\frac{{T}_{ref , pump}}{{T}_{pump}}\right)}^{α} ,$ where α≈ 0.5. For normal situations *f*(*Tₚᵤₘₚ, T_{ref,cuff}, T_{ref,pump}*) ≈ 1, resulting in most cases $\frac{dΔ p \left(t\right)}{d t} \approx \frac{{p}_{a}}{{p}_{ref , act}} {Q}_{max , ref , vol} ⋅ {a}_{v}$

As can be seen from formula (18) the gradient of the pressure difference scales with the ambient pressure. Furthermore, no information on the value or orifice properties is required. Hence, equation (18) shows that determining the ambient atmospheric pressure from the slope of the initial pressure gradient is possible. Fig. 14 shows an embodiment of the method 200 presented herein, wherein this knowledge is exploited. In a first step S202, a pressure difference measurement result (comprising information about pressure differences over time between the inside of the NIBP measurement system and the ambient atmosphere as measured by a gauge pressure sensor of the NIBP measurement system) is obtained. In a second step S2032, based on the obtained pressure difference measurement result a temporal pressure difference gradient is determined. In a third step S204 performance parameters of the pump, particularly *Q_{max,ref,vol},* are determined. In a fourth step, properties of the user's body part, to which the actuator/cuff 56 is attached, are determined. These properties particularly refer to the parameter *aᵥ*. In the last step S208 the ambient atmospheric pressure is determined based on a predetermined mathematical model of the obtained information.

As explained, this method to work, knowledge of *aᵥ* is important, which generally is not known upfront. However, it is possible to determine the parameter *aᵥ* from a steady state pressure difference measurement, for example, as this measurement gives us the atmospheric pressure, without a need to know the properties of the actuator pressure versus volume. From the measured ambient pressure it is possible to determine the parameter *aᵥ* from the measured initial gradient of the pressure and equation (18). Note that the steady state pressure method requires (see e.g. Fig. 11) to know effective valve area properties.

Hence as a follow-up it may also be possible to measure the initial pressure gradient and the belonging atmospheric pressure and determine from the relative change of the gradient a relative change in ambient pressure.

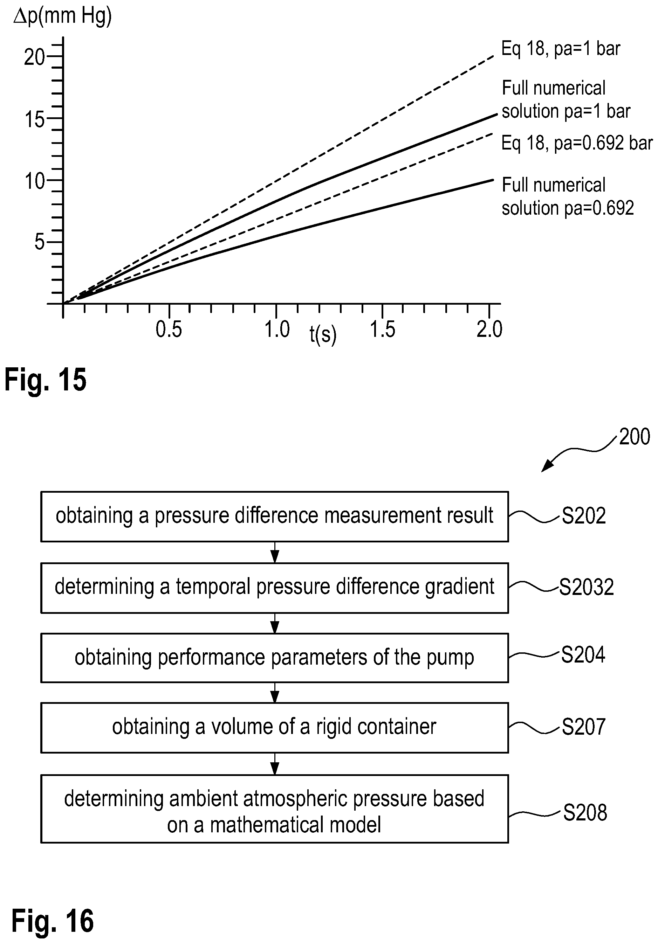

Comparing the solution of the full problem (see eq. (13) above) and the simplified solution of equation (18) reveals good agreement for small Δp. This can be seen from the diagram shown in Fig. 15.

Fig. 15 shows a diagram of (the increase of) the pressure difference with time for the simple solution according to equation (15) (dashed line) and the full numerical solution (solid line) at two different ambient pressure conditions, wherein *Q_{max,ref}=* 49.8 ml/s. *aᵥ* = 2.74·10⁷ Pa/m³ and *p_{ref,act} =* 0.963 atm.

Fig. 16 shows a flow chart of a fourth embodiment of the method 200 presented herein. To determine the ambient pressure, the pump (and possibly a tube) are mounted on a rigid box (or other container) with a known, fixed volume in this embodiment. For an operating pump, the pressure increase in the box is described by (see eq.(13) above) $\frac{d Δ p}{dt} = \frac{{T}_{b}}{{T}_{p}} \frac{{p}_{a} + Δ p}{\left({V}_{b}+{V}_{t}\right)} {Q}_{max , ref} \left(T,{p}_{a},ΔV,pwm%\right) ,$ which for small Δ*p* can be written as: $\frac{d Δ p}{dt} = \frac{{T}_{b}}{{T}_{p}} \frac{{p}_{a}}{\left({V}_{b}+{V}_{t}\right)} {Q}_{max , ref , vol} {\left(\frac{{T}_{ref , pump}}{{T}_{pump}}\right)}^{α}$

For most cases this equation can be simplified to $\frac{d Δ p}{dt} \approx \frac{{p}_{a}}{\left({V}_{b}+{V}_{t}\right)} {Q}_{max , ref , vol} ,$ wherein *V_{b}* and *Vₑ* are the box and tube volume. Hence, by increasing the pressure in a box of known size, the ambient atmospheric pressure can likewise be determined. In detail, the method 200 may comprise the following steps:

In a first step S202 of the method, a pressure difference measurement result (comprising information about pressure differences over time between the inside of the NIBP measurement system and the ambient atmosphere as measured by a gauge pressure sensor of the NIBP measurement system) is obtained. In a second step S2032, based on the obtained pressure difference measurement result a temporal pressure difference gradient is determined. In a third step S204 performance parameters of the pump are determined. However, contrary to the fourth step of the embodiment of the method 200 presented in Fig. 14, in this embodiment, in the fourth step S207 properties (particularly the volume) of the box (or other rigid container) are obtained. In the last step S208 the ambient atmospheric pressure is determined based on a predetermined mathematical model of the obtained information.

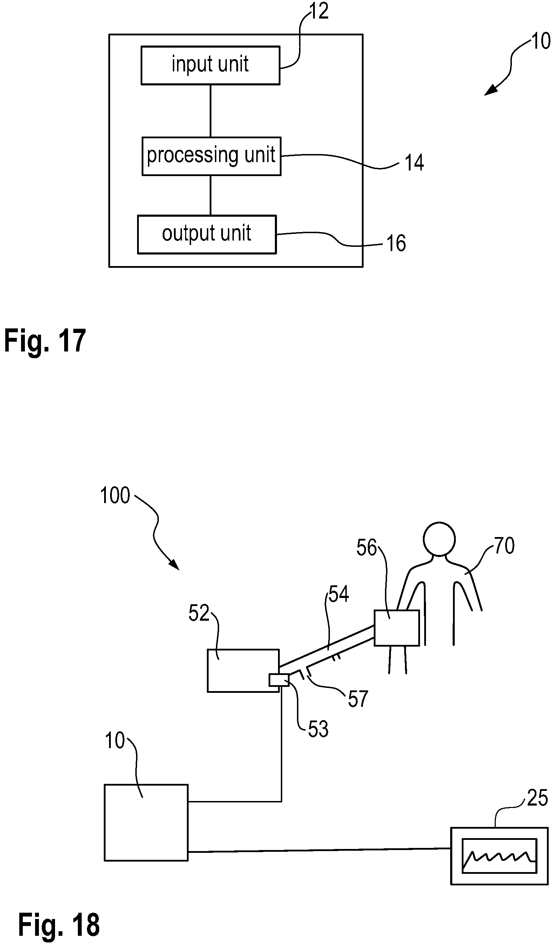

Fig. 17 shows an embodiment of a device 10 for determining ambient atmospheric pressure of a non-invasive blood pressure, NIBP, measurement system, according to the present invention. The device 10 comprises an input unit 12, a processing unit 14 and an output unit 16. In general, the device 10 needs not comprise an output unit 16.

The input unit 12 is configured to obtain a pressure difference measurement result comprising information about pressure differences over time between the inside of the NIBP measurement system and the ambient atmosphere as measured by a gauge pressure sensor of the NIBP measurement system. The input unit 12 may be directly coupled or connected to the gauge pressure sensor 53 of an NIBP measurement system 50 or may obtain (i.e. retrieve or receive) the pressure difference measurement result from a storage, buffer, network, or bus, etc. The input unit 12 may thus be communication interface or data interface, e.g., wired or wireless, such as a Bluetooth interface, WiFi interface, LAN interface, HDMI interface, direct cable connect, or any other suitable interface allowing signal transfer to the device 10.

The processing unit 14 is configured to determine the ambient atmospheric pressure based on a predetermined mathematical model, wherein the predetermined mathematical model is based on the obtained pressure difference measurement result, performance parameters of the (pump of the) NIBP measurement system and one or more of an effective opening area of a valve and/or orifice of the NIBP measurement system if a valve and/or orifice of the NIBP measurement system is opened, properties of a body part of a user if an actuator of the NIBP system is attached to the body part, and a volume of a rigid container if the NIBP measurement system comprises the rigid container, the rigid container at least replacing the actuator of the NIBP system. The processing unit 14 may be any kind of means configured to process the obtained information. It may be implemented in software and/or hardware, e.g. programmed processor or computer or app on a user device such as a tablet, laptop, PC, workstation, etc.

The output unit 16 may be configured to output information about the determined ambient atmospheric pressure. The output 16 may generally be any interface for outputting the information. It may transmit the information to a display unit or may provide it for retrieval by such unit. It may thus generally be any (wired or wireless) communication or data interface.

Fig. 18 shows an embodiment of a system 100 according to the present invention. The system 100 comprises an NIBP measurement system 50, a device 10 according to the present invention and a display unit 25.

The NIBP measurement system 50 comprises a pump 52 for pumping fluid from the ambient atmosphere to the inside of the NIBP measurement system 50, particularly (via the hose 54) to the actuator 56 wrapped around the arm of the user 70. The hose 54 comprises a valve 57 and an orifice 59, both having an effective opening area for releasing the fluid from the pump to the ambient atmosphere. The NIBP measurement system 50 further comprises a gauge pressure sensor 53 configured to measure pressure differences between the inside of the NIBP measurement system 50 and the ambient atmosphere. Measurement results of the gauge pressure sensor 53 are provided to the device 10 for determining the ambient atmospheric. Data related to the ambient atmospheric pressure may be provided by the device 10 to the display unit 25 for providing the ambient atmospheric pressure.

However, in general such data need not the output (and provided to a display unit 25 or the like), but may be used for further processing, for example, for enhancing an NIBP measurement.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Method (200) for determining ambient atmospheric pressure of a non-invasive blood pressure, NIBP, measurement system (50), the method (200) comprising:
- obtaining a pressure difference measurement result comprising information about pressure differences over time between the inside of the NIBP measurement system (50) and the ambient atmosphere as measured by a gauge pressure sensor (53) of the NIBP measurement system (50); and
- determining the ambient atmospheric pressure based on a predetermined mathematical model, wherein the predetermined mathematical model is based on the obtained pressure difference measurement result, performance parameters of a pump of the NIBP measurement system and one or more of:
- an effective opening area of a valve (57) and/or orifice (59) of the NIBP measurement system (50) if a valve (57) and/or orifice (59) of the NIBP measurement system (50) is opened,
- properties of a body part of a user and an actuator (56) of the NIBP measurement system (50) if the actuator (56) is attached to the body part, and
- a volume of a rigid container if the NIBP measurement system (50) comprises the rigid container, the rigid container at least replacing the actuator of the NIBP measurement system (50).

2. Method (200) according to claim 1, further comprising, if the valve (57) and/or orifice (59) is opened, determining a steady state pressure difference from the obtained measurement result, wherein the steady-state pressure difference is represented by the pressure difference measurement result if the NIBP measurement system (50) fails to comprise an actuator (56), and/or wherein the steady-state pressure difference is determined based on a function of time of the pressure differences if the NIBP measurement system (50) comprises an actuator (56), wherein the predefined mathematical model comprises a function and/or a look-up table linearly relating the ambient atmospheric pressure to the steady-state pressure difference.

3. Method (200) according to claim 2, further comprising determining whether the function is an appropriate mathematical model by determining whether the ratio of the steady-state pressure difference and the ambient atmospheric pressure is higher than 0.005 and equal or smaller than 0.15, particularly whether the ratio of the steady-state pressure difference and the ambient atmospheric pressure is higher than 0.02 and equal or smaller than 0.1.

4. Method (200) according to claim 2 or 3, further comprising determining one or more further steady-state pressure differences, and determining a mean steady-state pressure difference by averaging the plurality of steady-stated differences, wherein the ambient atmospheric pressure is determined based on the mean steady-state pressure difference.

5. Method (200) according to any one of the preceding claims, wherein the effective opening area of the valve (57) and/or orifice (59) is determined by measuring a deflation time of the valve (57) and/or orifice (59) for reducing an initial pressure to a final pressure in a predefined deflation volume and by relating the measured deflation time to the effective valve (57) and/or orifice (59) diameter based on a predetermined lookup table.

6. Method (200) according to claim 1, further comprising, if the actuator (56) of the NIBP measurement system (50) is attached to the body part and/or if the NIBP measurement system (50) comprises the rigid container, determining a temporal pressure difference gradient based on the obtained pressure difference measurement result, wherein the ambient atmospheric pressure is determined based on the temporal pressure difference gradient and the predefined mathematical model.

7. Method (200) according to claim 6, wherein the predefined mathematical model comprises a function and/or a look-up table, linearly relating the ambient atmospheric pressure to the temporal pressure difference gradient.

8. Method (200) according to any one of the preceding claims, wherein the performance parameters of the pump of the NIBP measurement system are functions of one or more of the ambient atmospheric pressure, the ambient atmospheric temperature, a voltage applied to the pump (52), and pulse width modulation percentage of a motor of the pump (52).

9. Method (200) according to any one of the preceding claims, wherein the performance parameters of the pump of the NIBP measurement system (50) are determined based on reference performance parameters of the pump of the NIBP measurement system (50) at reference ambient atmospheric pressure and reference ambient atmospheric temperature.

10. Method (200) according to claim 8 or 9, wherein the ambient atmospheric temperature is obtained from a temperature sensor of the NIBP measurement system (50).

11. Method (200) according to any one of the preceding claims, further comprising determining a flow rate and/or volume supplied to an actuator of the non-invasive blood pressure measurement system based on the determined ambient atmospheric pressure.

12. Device (10) for determining ambient atmospheric pressure of a non-invasive blood pressure, NIBP, measurement system (50), the device comprising:
- an input unit (12) configured to obtain a pressure difference measurement result comprising information about pressure differences over time between the inside of the NIBP measurement system (50) and the ambient atmosphere as measured by a gauge pressure sensor of the NIBP measurement system (50); and
- a processing unit (16) configured to determine the ambient atmospheric pressure based on a predetermined mathematical model, wherein the predetermined mathematical model is based on the obtained pressure difference measurement result, performance parameters of a pump of the NIBP measurement system (50) and one or more of:
- an effective opening area of a valve and/or orifice of the NIBP measurement system (50) if a valve (57) and/or orifice (59) of the NIBP measurement system (50) is opened,
- properties of a body part of a user and an actuator (56) of the NIBP measurement system (50) if the actuator (56) is attached to the body part, and
- a volume of a rigid container if the NIBP measurement system (50) comprises the rigid container, the rigid container at least replacing the actuator of the NIBP measurement system (50).

13. System (100) for determining ambient atmospheric pressure of a non-invasive blood pressure, NIBP, measurement system (50), the system (100) comprising:
- the NIBP measurement system (50), comprising:
- a pump (52) configured to pump fluid from an ambient atmosphere to the inside of the NIBP measurement system (50),
- a gauge pressure sensor (53) configured to measure pressure differences between the inside of the NIBP measurement system (50) and the ambient atmosphere, and
- one or more of:
- a valve (57) and/or orifice (59) comprising an effective opening area for releasing the fluid from the pump to the ambient atmosphere,
- an actuator (56) configured to be attached to a body part of a user, and
- a rigid container configured to at least replace the actuator (56); and
- a device (10) as claimed in claim 12.

14. System (100) according to claim 13, further comprising a temperature sensor configured to measure ambient temperature.

15. Computer program comprising program code means for causing a computer to carry out the steps of the method as claimed in any of claims 1 to 11 when said computer program is carried out on the computer.
